(19) Europäisches Patentamt / European Patent Office / Office européen des brevets

(11) **EP 2 448 465 B1**

(12) **FASCICULE DE BREVET EUROPEEN**

(45) Date de publication et mention
de la délivrance du brevet:
**10.09.2014 Bulletin 2014/37**

(21) Numéro de dépôt: **10745338.3**

(22) Date de dépôt: **25.06.2010**

(51) Int Cl.:
***A61B 3/028*** *(2006.01)*

(86) Numéro de dépôt international:
**PCT/FR2010/051319**

(87) Numéro de publication internationale:
**WO 2011/001082 (06.01.2011 Gazette 2011/01)**

(54) **CARACTERISATION D'UNE PERCEPTION DE FLOU**

KENNZEICHNUNG DER WAHRNEHMUNG EINER UNSCHÄRFE

CHARACTERIZATION OF A PERCEPTION OF BLUR

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **29.06.2009 FR 0954408**

(43) Date de publication de la demande:
**09.05.2012 Bulletin 2012/19**

(73) Titulaire: **Essilor International
(Compagnie Générale d'Optique)
94220 Charenton-le-Pont (FR)**

(72) Inventeurs:
• **DROBE, Björn
F-94220 Charenton-le-Pont (FR)**
• **GIRAUDET, Guillaume
F-94220 Charenton-le-Pont (FR)**

(74) Mandataire: **Cabinet Plasseraud
52, rue de la Victoire
75440 Paris Cedex 09 (FR)**

(56) Documents cités:
**US-A- 4 365 873      US-A1- 2004 174 499**

• **AKUTSU H; BEDELL H E; PATEL S S:
"Recognition thresholds for letters with
simulated dioptric blur", OPTOMETRY AND
VISION SCIENCE, vol. 77, no. 10, octobre 2000
(2000-10), pages 524-530, XP8121215,**

EP 2 448 465 B1

**EP 2 448 465 B1**

**Description**

La présente invention concerne un procédé de caractérisation d'une perception de flou par un sujet, ainsi qu'un dispositif qui est adapté pour mettre en oeuvre un tel procédé.

De façon connue, une différence entre l'amétropie d'un porteur de lunettes et la correction ophtalmique qui est apportée par un verre de lunettes utilisé par ce porteur produit un brouillage de sa vision, appelé flou («blur» en anglais) dioptrique. Lorsque ce flou dioptrique résulte d'une défocalisation de l'image en arrière de la rétine, il est spontanément supprimé par une accommodation de l'oeil du porteur, au retard accommodatif («accommodation lag» en anglais) près et tant que la limite d'accommodation oculaire du porteur n'est pas dépassée. Il en résulte cependant une fatigue visuelle pour le porteur. D'une façon générale, en dehors de la faculté d'accommodation oculaire, le flou dioptrique constitue un défaut de la vision d'un porteur de lunettes.

Les verres de lunettes progressifs permettent à un porteur presbyte de voir clairement à des distances variables à travers une zone de vision de loin du verre, une zone de vision de près, et à travers un canal qui relie les zones de vision de loin et de près. Mais ils présentent en dehors de ces zones des variations de puissance optique et d'astigmatisme qui sont à l'origine de flou dioptrique pour le porteur. De tels verres progressifs sont alors conçus pour réaliser un compromis entre la largeur du champ visuel dans lequel la puissance optique et l'astigmatisme du verre correspondent à la prescription ophtalmique qui est établie pour le porteur, et un flou dioptrique qui reste limité pour des directions de regard qui traversent le verre en dehors des zones de vision de loin et de près. En particulier, le flou dioptrique à l'extérieur des zones de vision de loin et de près d'un verre progressif est d'autant plus élevé que ce verre possède une valeur d'addition qui est élevée. On rappelle que l'addition d'un verre progressif est la différence entre les valeurs de puissance optique de ce verre respectivement pour une direction de référence pour la vision de près et une direction de référence pour la vision de loin.

US2004/174499 **décrit un système pour déterminer la puissance optique d'un verre correcteur/d'une lentille de contact, qui est capable de déterminer la puissance optique d'un verre correcteur/d'une lentille de contact de façon appropriée pour un individu.**

Par ailleurs, de nombreuses études physiologiques ont montré que la perception visuelle du flou est très variable entre des sujets différents. Ainsi, deux porteurs de lunettes qui ont des prescriptions ophtalmiques identiques et qui sont équipés de verres de lunettes aussi identiques peuvent être gênés différemment par le flou dioptrique qui est produit par ces verres, pour certaines directions obliques de leur regard. Par exemple, un premier porteur peut se déclarer gêné par ce flou alors qu'un second porteur peut confirmer un bon confort visuel. Le compromis qui est réalisé par ces verres identiques entre leur fonction ophtalmique et le flou dioptrique résiduel qu'ils produisent est alors approprié pour le second porteur, et doit être modifié pour le premier. Il est donc nécessaire de tenir compte de la sensibilité de chaque porteur à la perception du flou lors de l'attribution d'un verre progressif à ce porteur.

Pour cela, un test est nécessaire, qui permette d'obtenir d'un sujet son appréciation de perception de flou dans des conditions identifiées. Il suffirait alors, en principe, de lui fournir un verre de lunettes qui produise des valeurs de flou dioptrique inférieures à une valeur-seuil pour laquelle il a déclaré que ce niveau de flou était acceptable pour lui. Mais plusieurs difficultés apparaissent alors pour la réalisation par le sujet d'un test de perception du flou :

- la netteté qui est perçue par le sujet pour une image qui est lui présentée dépend de son éloignement par rapport à cette image. En effet, plus le sujet est éloigné de l'image, moins des détails de celle-ci lui apparaissent flous car ils deviennent trop petits pour être visibles ;

- la perception du flou est en partie subjective, car elle peut être influencée par une sensation de contrainte ou de liberté de mouvement du sujet pendant le test. En particulier, la liberté pour le sujet de pouvoir reculer ou se rapprocher d'une image dont le flou est à évaluer est importante ;

- un test de perception d'un flou dioptrique réel consisterait pour le sujet à apprécier le flou qui résulterait d'une défocalisation d'un système optique à travers lequel il observerait une image. Or un tel test est peu commode à mettre en oeuvre et contraignant pour le sujet. En effet, pendant le test, le sujet serait contraint à regarder à travers le système optique, ce qui ne reproduit pas des conditions de vision de la vie courante. En particulier, son champ visuel serait limité transversalement par le système optique, ce qui constitue une contrainte susceptible d'altérer significativement sa perception du flou ; et

- le diamètre pupillaire intervient involontairement dans la perception du flou, puisqu'il détermine une section transversale des faisceaux lumineux qui convergent imparfaitement sur la rétine à partir d'un point de l'image regardée. Or ce diamètre pupillaire varie en fonction de la luminosité ambiante.

Or, l'adoption d'une valeur-seuil trop basse pour le flou dioptrique, en dessous de laquelle le flou est considéré comme acceptable pour un futur porteur de verres de lunettes, est susceptible d'aboutir à une fourniture de verres de lunettes qui présenteraient d'autres caractéristiques dégradées inutilement dans un compromis entre le flou et ces autres caractéristiques. Par exemple, la qualité de vision dynamique à travers un verre de lunettes progressif peut être abaissée, pour obtenir un flou dioptrique qui reste faible dans des champs du verre qui sont plus larges.

La présente invention vise alors à répondre notamment aux buts suivants.

Un premier but de l'invention est de fournir un test de perception de flou par un sujet qui fournisse un résultat fiable.

Un deuxième but de l'invention consiste à obtenir une appréciation par le sujet du flou qu'il perçoit lorsqu'il regarde une image, d'une façon qui soit cohérente avec une détermination optique de ce flou.

Un troisième but de l'invention est de fournir un test de la perception du flou par le sujet, dont le résultat soit représentatif d'une sensation de flou perçu qu'apprécierait le sujet dans la vie courante.

Un quatrième but de l'invention est de fournir un test de perception de flou qui soit facile à mettre en oeuvre et peu onéreux.

Pour cela, l'invention propose un procédé de caractérisation d'une perception de flou par un sujet, comprenant les étapes suivantes :

/1/ sélectionner un critère de perception du flou qui permette d'établir une limite de flou à partir de laquelle le critère est satisfait ou non pour le sujet ;

/2/ générer puis afficher une image sur un écran de présentation, cette image ayant un flou qui correspond à un filtrage passe-bas de composantes d'image en fonction de fréquences spatiales de ces composantes d'image ;

/3/ pour le sujet, alors qu'il est libre de s'éloigner ou de se rapprocher de l'écran de présentation en variant une distance d'observation : observer l'image affichée et indiquer si le critère du perception de flou sélectionné à l'étape /1/ est satisfait ou non ;

/4/ sélectionner une valeur de la distance d'observation qui est réalisée par le sujet à au moins un instant pendant l'étape /3/ ;

/5/ à partir de la valeur sélectionnée à l'étape /4/ pour la distance d'observation, d'une fréquence spatiale de coupure du filtrage passe-bas de l'image affichée et éventuellement d'une valeur de diamètre pupillaire, déterminer une valeur d'un flou dioptrique pour l'image affichée lorsqu'elle est observée par le sujet, cette valeur étant associée à l'indication fournie par le sujet à l'étape /3/ en réponse au critère de perception de flou.

En outre, la valeur du flou dioptrique qui est déterminée à l'étape /5/ est une fonction décroissante de la valeur qui est sélectionnée pour la distance d'observation lorsque la fréquence spatiale de coupure est constante, pour le filtrage passe-bas de l'image affichée.

Ainsi, dans un procédé selon l'invention, la valeur du flou dioptrique est déterminée à partir d'une fréquence spatiale de coupure du filtrage passe-bas des composantes de l'image affichée, et d'une valeur de la distance d'observation qui est adoptée par le sujet. Optionnellement, une valeur de son diamètre pupillaire peut aussi être prise en compte. De cette façon, le procédé fournit une appréciation par le sujet de sa perception du flou qui est corrélée à une valeur de flou dioptrique établie en tenant compte des conditions du test de perception. Autrement dit, l'appréciation de la perception du flou de l'image par le sujet est affectée à une valeur de quantification du flou qui possède un sens optique. Il est alors possible de comparer la valeur de flou dioptrique qui est déterminée par un procédé selon l'invention pour un sujet, à des valeurs de flou dioptrique qui sont calculées pour un verre de lunettes, ou à une valeur de flou dioptrique qui est déterminée pour un autre sujet en utilisant aussi un procédé conforme à l'invention.

En particulier, la valeur du flou dioptrique qui est déterminée à l'étape /5/ peut être inversement proportionnelle à la valeur qui est sélectionnée à l'étape /4/ pour la distance d'observation, lorsque la fréquence spatiale de coupure du filtrage passe-bas de l'image affichée est constante.

Le filtrage passe-bas de l'image affichée est caractérisé par la fréquence spatiale de coupure des composantes de l'image, lorsque cette image est décomposée en une combinaison de telles composantes qui reconstituent ensemble l'image affichée. Dans ce cas, la valeur du flou dioptrique qui est déterminée à l'étape /5/ peut être inversement proportionnelle à la fréquence spatiale de coupure du filtrage passe-bas de l'image affichée, lorsque la valeur qui est sélectionnée à l'étape /4/ pour la distance d'observation est constante. En particulier, elle peut être calculée en utilisant la formule suivante :

$$B(dioptrie) = K / [DO(mètre) \times FC(mètre^{-1})] \qquad (1)$$

où B est la valeur du flou dioptrique exprimée en dioptries, DO est la valeur de la distance d'observation exprimée en mètres, FC est la fréquence spatiale de coupure du filtrage passe-bas de l'image affichée, exprimée en cycles par mètre mesuré parallèlement à l'écran, et K est un coefficient de proportionnalité positif et non nul.

En outre, étant donné que le sujet est libre de se déplacer pour regarder l'image affichée, il fournit l'indication sur sa perception du flou dans l'image qui est affichée, avec des conditions qui sont similaires à celles de la vie courante. En particulier, le procédé du test de perception du flou n'introduit pas ou peu de contraintes supplémentaires par rapport à des conditions usuelles de vision, qui seraient susceptibles de modifier son appréciation de sa perception du flou. Notamment, le champ de vision du sujet n'est pas limité transversalement, comme le ferait un instrument optique qui serait utilisé pour regarder l'image.

Enfin, l'image qui est affichée peut être générée par une unité informatique, par exemple en utilisant un filtrage numérique. L'écran d'affichage peut alors être commandé par cette unité informatique. D'une façon générale, le procédé de l'invention peut être réalisé en utilisant des dispositifs courants, peu onéreux et peu encombrants. De plus, il est assez court à réaliser pour le sujet. Il est donc particulièrement adapté pour être mis en oeuvre dans une boutique de vente au détail de verres de lunettes ophtalmiques, telle qu'une boutique d'opticien.

Le critère de perception du flou qui est utilisé dans un procédé conforme à l'invention permet de caractériser la perception du flou telle qu'elle est appréciée par le sujet. Par exemple, ce peut être un critère de détection de flou, un critère de gêne visuelle causée par le flou, ou un critère de perte de lisibilité d'au moins un caractère alphanumérique qui est contenu dans l'image affichée.

Enfin, la valeur du diamètre pupillaire qui est éventuellement utilisée à l'étape /5/ peut résulter d'une mesure effectuée sur le sujet.

L'invention propose en outre un dispositif qui est adapté pour caractériser une perception de flou par un utilisateur, ce dispositif comprenant :

- un système de génération d'images qui ont chacune un flou correspondant à un filtrage passe-bas de composantes d'image en fonction de fréquences spatiales de ces composantes d'image ;

- un écran, qui est adapté pour afficher les images générées par le système de génération d'images ;

- une unité de traitement de données, qui est adaptée pour déterminer une valeur d'un flou dioptrique à partir d'une valeur de distance d'observation adoptée par l'utilisateur pour observer une image affichée sur l'écran, d'une fréquence spatiale de coupure du filtrage passe-bas de l'image affichée, et éventuellement d'une valeur de diamètre pupillaire.

Un tel dispositif peut être utilisé pour caractériser la perception du flou par un sujet selon un procédé tel que décrit précédemment. En outre, il peut être compact et peu onéreux.

D'autres particularités et avantages de la présente invention apparaîtront dans la description ci-après d'exemples de mise en oeuvre non limitatifs, en référence aux figures annexées qui sont présentées maintenant :

- la figure 1 illustre une mise en oeuvre d'une caractérisation de perception de flou selon l'invention ; et

- la figure 2 est un diagramme synoptique des étapes d'un procédé selon l'invention.

Un dispositif de caractérisation d'une perception de flou par un sujet 10 peut comprendre une unité informatique 21 et un écran 20. Avantageusement, l'unité 21 est adaptée pour sélectionner et/ou calculer des images, commander un affichage de ces images sur l'écran 20, et déterminer une valeur de flou dioptrique qui correspond à l'image affichée en tenant compte de paramètres d'observation, dont la distance d'observation entre le sujet 10 et l'écran 20. De préférence, l'écran 20 est situé à hauteur des yeux du sujet 10.

Avantageusement, le dispositif comprend en outre un système de mesure de distance, qui est agencé pour mesurer la distance d'observation, notée DO sur la figure. Tous les systèmes de mesure de distance peuvent être utilisés, mais le système de mesure de distance qui est utilisé est de préférence adapté pour mesurer la distance d'observation DO sans liaison matérielle continue entre l'écran 20 et l'utilisateur 10, et éventuellement selon un enchaînement continu de mesures. De cette façon, la distance d'observation DO peut être mesurée pendant le procédé sans créer de contraintes pour le sujet 10. En outre, elle peut être mesurée sans que le sujet 10 en soit averti, pour qu'il soit encore plus libre physiquement et psychologiquement dans ses déplacements par rapport à l'écran 20. Les systèmes suivants permettent notamment une mise en oeuvre simple et confortable de l'invention :

- une graduation qui peut être inscrite sur le sol à partir d'une direction verticale passant par l'écran 20, et parallèlement à laquelle le sujet 10 peut se déplacer en marchant ou en reculant, pour se rapprocher ou s'éloigner de l'écran 20 ;

- un système de télémétrie à base d'ultrasons, de rayonnement infrarouge ou d'un faisceau laser, qui peut comprendre un émetteur et/ou un capteur disposé au niveau de l'écran 20 en direction du sujet 10 ; ou

- un système optique de mesure de distance.

De tels systèmes de mesure de distance sont bien connus de l'Homme du métier, de sorte qu'il n'est pas nécessaire de les décrire ici. Eventuellement, certains de ces systèmes peuvent comprendre un ou plusieurs émetteurs, capteurs ou réflecteurs qui sont portés par le sujet 10. De tels émetteurs/capteurs/réflecteurs peuvent notamment être incorporés dans une monture de lunettes 11 dont le sujet 10 est équipé. Le système de mesure de la distance d'observation DO peut être relié à l'unité informatique 21, de façon que le résultat de chaque mesure puisse être directement utilisé pour déterminer la valeur du flou dioptrique.

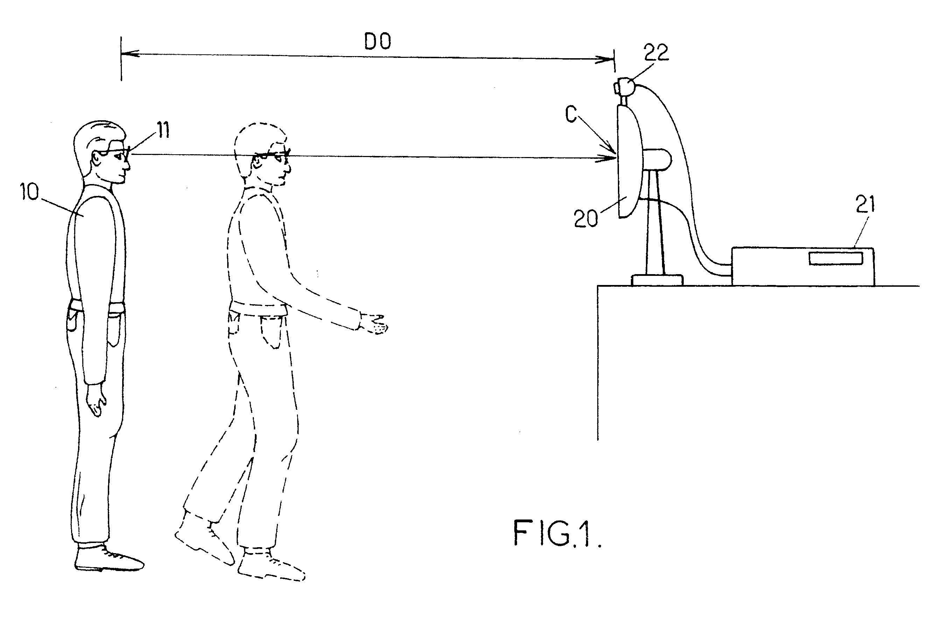

D'une façon facultative, le dispositif de caractérisation de la perception du flou peut comprendre en outre un système de mesure d'un diamètre pupillaire de l'utilisateur 10. De tels systèmes qui produisent des mesures du diamètre de l'une au moins des pupilles du sujet 10 sont aussi connus de l'Homme du métier. Ils peuvent être incorporés dans la paire de lunettes 11 dont le sujet 10 peut être équipé, ou bien comprendre une caméra qui produit à distance une image grossie de l'un au moins des yeux du sujet 10. Dans ce dernier cas, un traitement d'image peut alors identifier automatiquement chaque pupille du sujet 10 dans les images saisies, et déterminer en temps réel un diamètre de cette pupille. Le diamètre pupillaire est noté DP dans la suite. Avantageusement, le système de mesure de distance et le système de mesure du diamètre pupillaire peuvent être adaptés pour réaliser simultanément les mesures de la distance d'observation DO et du diamètre pupillaire DP de l'utilisateur 10, à un même instant. Dans la mise en oeuvre de l'invention qui est illustrée par la figure 1, les deux systèmes de mesure sont combinés en utilisant une caméra vidéo 22 qui est disposée sur l'écran 20, et des réflecteurs appropriés qui sont portés par la monture de la paire de lunettes 11.

Lorsque le dispositif de caractérisation de la perception du flou ne comprend pas de système de mesure du diamètre pupillaire de l'utilisateur 10, une valeur fixe du diamètre DP peut être adoptée, par exemple égale à 2 mm (millimètre). Dans ce cas notamment, la luminosité ambiante ainsi que la luminosité des images qui sont affichées sur l'écran 20 sont avantageusement fixées pour réduire des variations du diamètre pupillaire du sujet 10 pendant la caractérisation de sa perception du flou.

Il n'est pas indispensable que les lunettes 11 dont le sujet 10 peut être équipé pendant le déroulement du procédé produisent une correction d'amétropie. Une telle correction d'amétropie peut être absente lorsque l'amétropie qui est diagnostiquée pour le sujet 10 est elle-même faible. A l'inverse, la correction d'une partie au moins de l'amétropie du sujet 10 par les lunettes 11, pendant la caractérisation de sa perception du flou, peut être nécessaire lorsque l'amétropie du sujet 10 est importante. Dans ce cas, les verres de la paire de lunettes 11 sont de préférence unifocaux. La correction d'amétropie qui est réalisée pour le sujet 10 pendant la caractérisation de sa perception du flou pourra alors être déduite de la valeur du flou dioptrique qui est déterminée à partir de l'image affichée sur l'écran 20, de la distance d'observation DO et éventuellement du diamètre pupillaire DP.

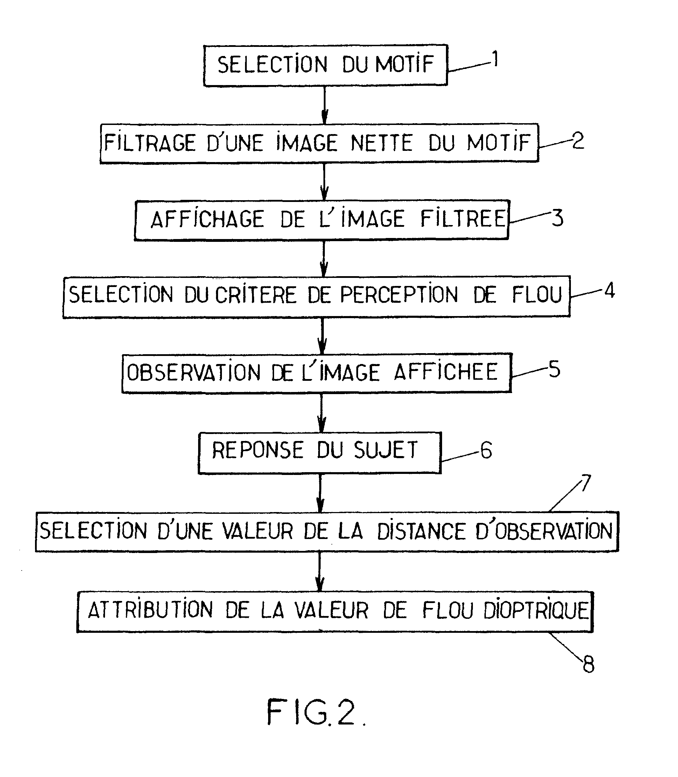

Le déroulement de la caractérisation de la perception du flou par le sujet 10 est maintenant décrit, en référence à la figure 2.

Une image est générée, qui comporte un motif affecté d'un flou d'affichage. Le motif est sélectionné en premier (étape 1), et peut être un caractère alphanumérique C. Le flou d'affichage est introduit ensuite dans l'image en réalisant un filtrage passe-bas d'une image nette du motif C (étape 2). Un tel filtrage peut être réalisé de façon analogique au niveau des signaux d'affichage sur l'écran 20. De préférence, il est réalisé numériquement par l'unité informatique 21 à partir des données d'affichage du motif C.

Un tel filtrage passe-bas est équivalent à une décomposition de l'image nette du motif C en composantes d'images qui correspondent à des fréquences spatiales différentes, suivie d'une réduction des amplitudes des composantes d'image qui ont des fréquences spatiales élevées. Le principe d'un tel filtrage est connu, et notamment que le filtrage peut présenter un coefficient d'atténuation des composantes d'image qui varie de différentes façons en fonction de la fréquence spatiale. L'image qui est affichée est la recombinaison des composantes d'image ainsi atténuées. Le motif C présente alors un contour diffus dans l'image affichée, à travers lequel le contraste varie progressivement à partir de l'intérieur du motif vers l'extérieur du motif, produisant un flou d'affichage. Un tel flou est réel dans l'image affichée, par opposition au flou dioptrique qui est produit dans l'oeil lorsque le sujet regarde une image nette. L'un des buts de l'invention consiste à établir une relation entre le flou réel d'une image qui est généré volontairement, et le flou dioptrique. En effet, un flou d'affichage est plus facile à produire de façon contrôlée qu'un flou qui résulte d'un écart de mise au point d'un instrument optique.

De façon connue, un filtrage passe-bas qui est appliqué à une image peut être caractérisé par une fréquence spatiale de coupure. Les composantes d'image sont alors atténuées lorsque leurs fréquences spatiales respectives sont supérieures à la fréquence spatiale de coupure, et ne sont pas significativement atténuées lorsque leurs fréquences spatiales respectives sont inférieures à la fréquence spatiale de coupure. La fréquence spatiale de coupure détermine alors la distance minimale dans l'image filtrée, pour la séparation entre deux détails du motif C qui peuvent être identifiés séparément l'un de l'autre. En dessous de cette distance minimale de séparation, les détails apparaissent confondus dans l'image filtrée.

La décomposition de l'image en composantes d'image qui sont identifiées par leurs fréquences spatiales respectives peut être une transformation de Fourier bidimensionnelle. Mais d'autres types de décompositions d'image peuvent être utilisés alternativement, tels que des décompositions en ondelettes («wavelets» en anglais), connues de l'Homme du métier.

L'image filtrée peut être soit calculée en temps réel pendant que le sujet 10 réalise la caractérisation de sa perception du flou selon l'invention, soit avoir été calculée préalablement puis enregistrée dans une bibliothèque d'images pour différentes valeurs de la fréquence spatiale de coupure du filtrage passe-bas. Dans ce second cas, l'image qui est affichée est sélectionnée dans la bibliothèque en fonction du motif et du filtrage.

L'image filtrée du motif C est affichée sur l'écran 20 (étape 3).

Le sujet 10 se place devant l'écran 20. Un opérateur lui demande alors de regarder l'image affichée et d'indiquer un niveau du flou avec lequel il distingue visuellement le motif C. Ce niveau de flou est évalué conformément à un critère de perception de flou qui est sélectionné par l'opérateur et communiqué au sujet 10 (étape 4). Ce critère peut être l'un des suivants, qui ne sont donnés qu'à titre d'exemples :

- du flou est-il perceptible ou non par le sujet 10 dans l'image affichée ?

- le flou cause-t-il une gêne visuelle au sujet 10, lors de l'observation de l'image affichée ?

- le caractère alphanumérique C est-il lisible pour le sujet 10 ?

Ces trois critères sont donc, dans l'ordre : une détection du flou, une gêne ressentie et une perte de lisibilité. Eventuellement, ces critères de perception du peuvent être complétés par d'autres pour obtenir une caractérisation plus fine de la perception du flou par le sujet 10.

Le sujet 10 est libre de se rapprocher ou de s'éloigner de l'écran 20 lorsqu'il observe l'image affichée (étape 5). De cette façon, il peut se placer à une distance de l'écran 20 qui correspond à une condition d'observation qui est habituelle pour lui. Il peut ainsi reproduire les conditions de vision qui sont les plus fréquentes dans sa vie quotidienne. Il peut aussi se placer à une distance de l'écran 20 qui correspond pour lui à une sensation de confort meilleur. En fait, le choix de la distance d'observation DO par le sujet 10 est aussi influencé inconsciemment par des paramètres environnementaux tels que les dimensions de la pièce dans laquelle le test de perception de flou est effectué, l'encombrement de cette pièce, la luminosité ambiante, la teinte des murs, des motifs qui sont présents sur les murs, un contraste de tels motifs muraux, la luminosité de l'image affichée, le motif et les teintes de cette image, etc. Pour cette raison, il est avantageux de sélectionner une méthode psychophysique de perception visuelle, qui permet de réduire l'influence de tels paramètres environnementaux sur le résultat de la caractérisation de perception du flou. De telles méthodes psychophysiques sont décrites dans des articles ou des ouvrages disponibles, de sorte qu'il n'est pas nécessaire de les présenter à nouveau ici.

La distance d'observation DO peut être mesurée continûment pendant que le sujet 10 observe l'image affichée.

Le sujet 10 répond alors la question de l'un au moins des critères de perception du flou, pour caractériser le niveau du flou qu'il perçoit dans l'image qui est affichée sur l'écran 20 (étape 6).

Une valeur de la distance d'observation DO est alors sélectionnée, qui a été adoptée par le sujet 10 (étape 7). Cette sélection peut être réalisée de plusieurs façons, parmi lesquelles :

- la valeur qui est sélectionnée peut correspondre à la plus courte distance d'observation qui a été réalisée par le sujet 10 à au moins un instant pendant son observation de l'image affichée ; et

- la valeur qui est sélectionnée peut correspondre à une distance d'observation qui est indiquée par le sujet 10 comme lui procurant un confort maximal d'observation de l'image affichée.

Une valeur du diamètre pupillaire DP peut aussi être sélectionnée. Ce peut être une valeur de référence, par exemple 2 mm, ou une valeur qui est mesurée sur le sujet 10. De préférence, le diamètre pupillaire DP est mesuré sur le sujet 10 continûment pendant qu'il observe l'image affichée, et la valeur de ce diamètre DP qui est sélectionnée peut correspondre à un même instant que la distance d'observation DO qui a aussi été sélectionnée.

L'invention permet alors d'attribuer une valeur de flou dioptrique à la situation d'observation de l'image affichée telle qu'elle est regardée par le sujet 10 (étape 8). Chaque réponse du sujet 10 à la question d'un des critères de perception du flou, lorsqu'elle est associée à la valeur du flou dioptrique, caractérise la perception du flou par le sujet 10.

De façon générale selon l'invention, cette valeur du flou dioptrique est une fonction décroissante de la distance d'observation DO qui a été sélectionnée, lorsque le flou dioptrique est exprimé en fonction de cette distance d'observation DO et de la fréquence spatiale de coupure FC. En effet, le contour diffus du motif C dans l'image affichée est d'autant moins visible par le sujet 10 que la distance d'observation DO est grande. En particulier, la valeur du flou dioptrique qui est déterminée selon l'invention peut être inversement proportionnelle à la valeur de la distance d'observation DO qui a été sélectionnée.

Cette valeur du flou dioptrique peut aussi être inversement proportionnelle à la fréquence spatiale de coupure FC du filtrage passe-bas de l'image affichée.

Dans ce cas, la valeur du flou dioptrique de l'image affichée, notée B, peut être déterminée selon une formule du type suivant :

$$B(dioptrie) = K / [DO(mètre) \times FC(mètre^{-1})] \qquad (1)$$

K étant un coefficient de proportionnalité positif non nul.

Par ailleurs, la fréquence spatiale de coupure FC de l'image filtrée est un nombre de cycles de variation d'intensité de composante d'image, par unité d'une longueur qui est mesurée parallèlement à l'écran 20. Elle peut être convertie en un nombre NC de cycles par degré d'écart angulaire de variation de la direction de regard du sujet 10, selon la formule suivante :

$$FC(mètre^{-1}) = NC(cycles/degré) \times 180 / (\pi \times DO) \qquad (2)$$

En combinant les formules (1) et (2), il vient :

$$B(dioptrie) = K' / NC(cycles/degré) \qquad (3)$$

où K' = K x $\pi$/180. Autrement dit, dans les conditions qui viennent d'être indiquées, le flou dioptrique est inversement proportionnel au nombre de cycles NC par degré d'angle de vue pour le sujet 10, et devient indépendant de la distance d'observation DO. Lorsque la constante K' est égale à 0,25, le nombre NC de cycles par degré d'angle de vue correspond à l'acuité visuelle du sujet, telle qu'elle résulte de la loi de Swaine pour un flou dioptrique B qui serait produit par une défocalisation sphérique.

Selon un perfectionnement de l'invention, le flou dioptrique peut être déterminé plus précisément en tenant compte en plus du diamètre pupillaire DP. Le flou dioptrique est alors une fonction croissante de la valeur du diamètre pupillaire, en plus de ses variations en fonction de la distance d'observation DO et de la fréquence spatiale de coupure FC. Lorsque ce flou est assimilé à une défocalisation sphérique, l'équation (1) peut être remplacée par la suivante, au premier ordre non nul de variation en fonction du diamètre pupillaire DP :

$$B(dioptrie) = K \times [1 + \alpha \cdot DP^2] / [DO(mètre) \times FC(mètre^{-1})] \qquad (1')$$

où $\alpha$ est un coefficient positif. Lorsque le diamètre pupillaire DP est exprimé en millimètres, $\alpha$ peut être pris égal à 0,055 mm$^{-2}$, par exemple.

Eventuellement, l'image qui est affichée, notamment le filtrage passe-bas qui est appliqué à celle-ci, peut être ajustée pendant que le sujet 10 se déplace en fixant cette image sur l'écran 20. Par exemple, la fréquence spatiale de coupure FC peut être modifiée en fonction du résultat de la mesure en temps réel de la distance d'observation DO. Une telle modification peut compenser au moins partiellement la variation du flou dioptrique qui résulte des déplacements du sujet 10. Par exemple, la fréquence spatiale de coupure FC qui est utilisée pour l'image affichée peut être modifiée en temps réel pendant que le sujet observe celle-ci, de façon à maintenir constant le produit de cette fréquence spatiale de coupure FC par la distance d'observation DO. Dans ce cas, la valeur du flou dioptrique qui est obtenue, et qui est associée à l'indication de perception du flou donnée par le sujet 10 selon le critère de perception sélectionné, est automatiquement compensée de l'influence de la distance d'observation DO.

De façon plus générale, le système de génération d'images 21 peut être adapté pour générer l'image qui va être affichée sur l'écran 20 d'une façon variable en fonction d'au moins un résultat de mesure choisi parmi la valeur de la distance d'observation DO et la valeur du diamètre pupillaire du sujet 10.

Enfin, les étapes de génération et d'affichage de l'image sur l'écran 20, d'observation de celle-ci par le sujet 10 en pouvant s'éloigner ou se rapprocher, de sélection d'une valeur pour la distance d'observation DO et de détermination d'une valeur du flou dioptrique peuvent être répétées pour le même critère de perception du flou. Autrement dit, la séquence de ces étapes peut être exécutée plusieurs fois en variant la fréquence spatiale de coupure du filtrage passe-bas de l'image affichée pour des exécutions successives de cette séquence d'étapes. Alors, lorsque le sujet 10 fournit des indications qui sont opposées en réponse au critère de perception du flou pour au moins deux exécutions de la séquence, un encadrement d'une valeur-seuil du flou dioptrique qui correspond à ce critère de perception peut être déduit pour le sujet 10 de ces indications, corrélées avec les valeurs du flou dioptrique correspondantes qui ont été déterminées. Cette valeur-seuil du flou dioptrique correspond à la limite entre des réponses positive et négative du sujet 10 au critère de perception. Elle peut alors être utilisée pour ajuster le flou dioptrique d'un verre de correction ophtalmique qui est fourni au sujet. Lorsque ce verre est du type verre progressif, la valeur-seuil du flou dioptrique obtenue pour le sujet peut être utilisée pour ajuster un design du verre fourni en fonction de la sensibilité du sujet au flou visuel.

Il est entendu que l'invention peut être mise en oeuvre en modifiant certains aspects de celle-ci par rapport à la description qui vient d'en être donnée, tout en gardant certains des avantages mentionnés. En particulier, il est entendu que les formules mathématiques qui ont été données peuvent être remplacées par des formules qui caractérisent des variations différentes, quoiqu'ayant des sens de variations identiques, sans que les objectifs et avantages de l'invention soient modifiés.

## Revendications

**1.** Procédé de caractérisation d'une perception de flou par un sujet (10), comprenant les étapes suivantes :

/1/ sélectionner un critère de perception du flou permettant d'établir une limite de flou à partir de laquelle le critère est satisfait ou non pour le sujet (10) ;

/2/ générer puis afficher une image (C) sur un écran de présentation (20), ladite image ayant un flou correspondant à un filtrage passe-bas de composantes d'image en fonction de fréquences spatiales desdites composantes d'image ;

/3/ pour le sujet (10), ledit sujet étant libre de s'éloigner ou de se rapprocher de l'écran de présentation (20) en variant une distance d'observation (DO) : observer l'image affichée (C) et indiquer si le critère de perception du flou sélectionné à l'étape /1/ est satisfait ou non ;

/4/ sélectionner une valeur de la distance d'observation (DO) réalisée par le sujet (10) à au moins un instant pendant l'étape /3/ ;

/5/ à partir de la valeur sélectionnée à l'étape /4/ pour la distance d'observation (DO), d'une fréquence spatiale de coupure du filtrage passe-bas de l'image affichée (C) et éventuellement d'une valeur de diamètre pupillaire, déterminer une valeur d'un flou dioptrique pour l'image affichée observée par le sujet (10), ladite valeur étant associée à l'indication fournie par le sujet à l'étape /3/ en réponse au critère de perception de flou,

et ladite valeur du flou dioptrique étant une fonction décroissante de la valeur sélectionnée pour la distance d'observation (DO) lorsque la fréquence spatiale de coupure du filtrage passe-bas de l'image affichée (C) est constante.

**2.** Procédé selon la revendication 1, suivant lequel la valeur du flou dioptrique déterminée à l'étape /5/ est inversement proportionnelle à la valeur sélectionnée à l'étape /4/ pour la distance d'observation (DO) lorsque la fréquence spatiale de coupure du filtrage passe-bas de l'image affichée (C) est constante, et éventuellement est en outre une fonction croissante de la valeur du diamètre pupillaire.

**3.** Procédé selon la revendication 1 ou 2, suivant lequel la valeur du flou dioptrique déterminée à l'étape /5/ est inversement proportionnelle à la fréquence spatiale de coupure du filtrage passe-bas de l'image affichée (C) lorsque la valeur sélectionnée à l'étape /4/ pour la distance d'observation (DO) est constante.

**4.** Procédé selon l'une quelconque des revendications précédentes, suivant lequel la distance d'observation (DO) de l'image affichée (C) par le sujet (10) est mesurée continûment pendant l'étape /3/.

**5.** Procédé selon l'une quelconque des revendications 1 à 4, suivant lequel la valeur sélectionnée à l'étape /4/ pour la distance d'observation (DO) correspond à la plus courte distance d'observation réalisée par le sujet (10) à au moins un instant pendant l'étape /3/.

**6.** Procédé selon l'une quelconque des revendications 1 à 4, suivant lequel la valeur sélectionnée à l'étape /4/ pour la distance d'observation (DO) est indiquée par le sujet (10) comme procurant un confort maximal d'observation de l'image affichée (C) audit sujet.

**7.** Procédé selon l'une quelconque des revendications précédentes, suivant lequel la séquence des étapes /2/ à /5/ est exécutée plusieurs fois en variant la fréquence spatiale de coupure du filtrage passe-bas de l'image affichée pour des exécutions successives de ladite séquence d'étapes, de façon que le sujet (10) fournisse des indications opposées à l'étape /3/ en réponse au critère de perception du flou pour au moins deux exécutions de ladite séquence d'étapes,
et suivant lequel un encadrement d'une valeur-seuil du flou dioptrique correspondant audit critère de perception, est déduit pour le sujet (10) des indications opposées fournies par ledit sujet et corrélées avec les valeurs du flou dioptrique déterminées lors des exécutions correspondantes de l'étape /5/.

**8.** Procédé selon l'une quelconque des revendications précédentes, suivant lequel la valeur du diamètre pupillaire utilisée à l'étape /5/ résulte d'une mesure effectuée sur le sujet (10).

**9.** Procédé selon la revendication 8, suivant lequel le diamètre pupillaire est mesuré sur le sujet (10) continûment pendant l'étape /3/, et suivant lequel la valeur dudit diamètre pupillaire utilisée à l'étape /5/ correspond à un même instant que la distance d'observation (DO) sélectionnée à l'étape /4/.

**10.** Dispositif adapté pour caractériser une perception de flou par un utilisateur (10), comprenant :

- un système de génération d'images (21) ayant chacune un flou correspondant à un filtrage passe-bas de composantes d'image en fonction de fréquences spatiales desdites composantes d'image ;
- un écran (20) adapté pour afficher les images générées par le système de génération d'images ;
- une unité de traitement de données adaptée pour déterminer une valeur d'un flou dioptrique à partir d'une valeur de distance d'observation (DO) adoptée par l'utilisateur (10) pour observer une image affichée (C) sur l'écran (20), d'une fréquence spatiale de coupure du filtrage passe-bas de l'image affichée, et éventuellement d'une valeur de diamètre pupillaire.

**11.** Dispositif selon la revendication 10, comprenant en outre un système de mesure de distance, agencé pour mesurer la distance d'observation (DO) entre l'utilisateur (10) et l'écran (20).

**12.** Dispositif selon la revendication 11, dans lequel le système de mesure de distance est adapté pour mesurer la distance d'observation (DO) sans liaison matérielle continue entre l'écran (20) et l'utilisateur (10), de préférence selon un enchaînement continu de mesures.

**13.** Dispositif selon l'une quelconque des revendications 10 à 12, comprenant en outre un système de mesure d'un diamètre pupillaire de l'utilisateur (10).

**14.** Dispositif selon la revendication 11 ou 12 ensemble la revendication 13, dans lequel le système de mesure de distance et le système de mesure du diamètre pupillaire sont adaptés pour réaliser simultanément les mesures de la distance d'observation (DO) et du diamètre pupillaire de l'utilisateur (10), à un même instant.

**15.** Dispositif selon l'une quelconque des revendications 11 à 14, dans lequel le système de génération d'images (21) est adapté pour générer l'image affichée sur l'écran (20) d'une façon variable en fonction d'au moins un résultat de mesure choisi parmi la valeur de la distance d'observation (DO) et la valeur du diamètre pupillaire de l'utilisateur (10).

**Patentansprüche**

**1.** Verfahren zum Charakterisieren einer unscharfen Wahrnehmung durch eine Person (10), das die folgenden Schritte umfasst:

/1/ Wählen eines Unschärfewahrnehmungskriteriums, das ermöglicht, eine Unschärfegrenze zu bilden, anhand derer das Kriterium für die Person (10) erfüllt ist oder nicht;

/2/ Erzeugen und dann Anzeigen eines Bildes (C) auf einem Präsentationsbildschirm (20), wobei das Bild eine Unschärfe hat, die einer Tiefpassfilterung von Bildkomponenten als Funktion räumlicher Frequenzen der Bildkomponenten entspricht;

/3/ für die Person (10), wobei sich die Person beliebig an den Präsentationsbildschirm (20) annähern oder von ihm entfernen kann, indem sie einen Beobachtungsabstand (DO) variiert: Beobachten des angezeigten Bildes (C) und Angeben, ob das im Schritt /1/ gewählte Unschärfewahrnehmungskriterium erfüllt ist oder nicht;

/4/ Auswählen eines Wertes des Beobachtungsabstands (DO), der von der Person (10) in wenigstens einer Instanz während des Schrittes /3/ verwirklicht worden ist;

/5/ anhand des im Schritt /4/ ausgewählten Wertes für den Beobachtungsabstand (DO), einer räumlichen Kappungsfrequenz der Tiefpassfilterung des angezeigten Bildes (C) und eventuell eines Pupillendurchmesserwertes, Bestimmen eines dioptrischen Unschärfewertes für das von der Person (10) beobachtete angezeigte Bild, wobei der Wert einer Angabe zugeordnet wird, die von der Person im Schritt /3/ in Antwort auf das Unschärfewahrnehmungskriterium gemacht wird,

wobei der dioptrische Unschärfewert eine abnehmende Funktion des gewählten Wertes für den Beobachtungsabstand (DO) ist, wenn die räumliche Kappungsfrequenz der Tiefpassfilterung des angezeigten Bildes (C) konstant ist.

2. Verfahren nach Anspruch 1, wobei der im Schritt /5/ bestimmte dioptrische Unschärfewert zu dem im Schritt /4/ gewählten Wert für den Beobachtungsabstand (DO) umgekehrt proportional ist, wenn die räumliche Kappungsfrequenz der Tiefpassfilterung des angezeigten Bildes (C) konstant ist, und eventuell außerdem eine zunehmende Funktion des Pupillendurchmesserwertes ist.

3. Verfahren nach Anspruch 1 oder 2, wobei der im Schritt /5/ bestimmte dioptrische Unschärfewert zu der räumlichen Kappungsfrequenz der Tiefpassfilterung des angezeigten Bildes (C) umgekehrt proportional ist, wenn der in Schritt /4/ gewählte Wert für den Beobachtungsabstand (DO) konstant ist.

4. Verfahren nach einem der vorhergehenden Ansprüche, wobei der Beobachtungsabstand (DO) des angezeigten Bildes (C) für die Person (10) während des Schrittes /3/ ununterbrochen gemessen wird.

5. Verfahren nach einem der Ansprüche 1 bis 4, wobei der im Schritt /4/ gewählte Wert für den Beobachtungsabstand (DO) dem kürzesten Beobachtungsabstand, der von der Person (10) in wenigstens einer Instanz während des Schrittes /3/ verwirklicht wird, entspricht.

6. Verfahren nach einem der Ansprüche 1 bis 4, wobei der im Schritt /4/ gewählte Wert für den Beobachtungsabstand (DO) von der Person (10) als einen maximalen Komfort für die Person bei der Beobachtung des angezeigten Bildes (C) herbeiführend angegeben wird.

7. Verfahren nach einem der vorhergehenden Ansprüche, wobei die Folge der Schritte /2/ bis /5/ mehrmals bei veränderter räumlicher Kappungsfrequenz der Tiefpassfilterung des angezeigten Bildes für die aufeinanderfolgenden Ausführungen der Folge von Schritten ausgeführt wird, derart, dass die Person (10) als Antwort auf das Unschärfewahrnehmungskriterium für wenigstens zwei Ausführungen der Folge von Schritten im Schritt /3/ entgegengesetzte Angaben macht,

und wobei ein Rahmen eines Schwellenwerts der dioptrischen Unschärfe, der dem Wahrnehmungskriterium entspricht, für die Person (10) mit den entgegengesetzten Angaben, die von der Person gemacht werden und die mit den dioptrischen Unschärfewerten, die in den entsprechenden Ausführungen des Schrittes /5/ bestimmt werden, korreliert werden, abgeleitet wird.

8. Verfahren nach einem der vorhergehenden Ansprüche, wobei sich der im Schritt /5/ verwendete Pupillendurchmesserwert aus einer an der Person (10) vorgenommenen Messung ergibt.

9. Verfahren nach Anspruch 8, wobei der Pupillendurchmesser an der Person (10) während des Schrittes /3/ ununterbrochen gemessen wird und wobei der Wert des im Schritt /5/ verwendeten Pupillendurchmessers derselben Instanz wie der Beobachtungsabstand (DO), der im Schritt /4/ gewählt wird, entspricht.

10. Vorrichtung, die dafür ausgelegt ist, eine Unschärfewahrnehmung durch einen Anwender (10) zu charakterisieren, die Folgendes umfasst:

- ein System (21) zum Erzeugen von Bildern, wovon jedes eine Unschärfe hat, die einer Tiefpassfilterung von Bildkomponenten als Funktion räumlicher Frequenzen der Bildkomponenten entspricht;
- einen Bildschirm (20), der dafür ausgelegt ist, die von dem Bilderzeugungssystem erzeugten Bilder anzuzeigen;
- eine Datenverarbeitungseinheit, die dafür ausgelegt ist, einen Wert einer dioptrischen Unschärfe anhand eines Wertes des Beobachtungsabstands (DO), der von dem Anwender (10) eingenommen wird, um ein angezeigtes Bild (C) auf dem Bildschirm (20) zu beobachten, einer räumlichen Kappungsfrequenz der Tiefpassfilterung des angezeigten Bildes und eventuell eines Wertes des Pupillendurchmessers zu bestimmen.

**11.** Vorrichtung nach Anspruch 10, die außerdem ein Abstandsmesssystem umfasst, das dafür ausgelegt ist, den Beobachtungsabstand (DO) zwischen dem Anwender (10) und dem Bildschirm (20) zu messen.

**12.** Vorrichtung nach Anspruch 11, wobei das Abstandsmesssystem dafür ausgelegt ist, den Beobachtungsabstand (DO) ohne ununterbrochene materielle Verbindung zwischen dem Bildschirm (20) und dem Anwender (10) vorzugsweise gemäß einer ununterbrochenen Messkette zu messen.

**13.** Vorrichtung nach einem der Ansprüche 10 bis 12, die außerdem ein System zum Messen des Pupillendurchmessers des Anwenders (10) umfasst.

**14.** Vorrichtung nach Anspruch 11 oder 12 in Verbindung mit Anspruch 13, wobei das System zum Messen des Abstands und das System zum Messen des Pupillendurchmessers dafür ausgelegt sind, die Messungen des Beobachtungsabstands (DO) und des Pupillendurchmessers des Anwenders (10) zum selben Zeitpunkt gleichzeitig auszuführen.

**15.** Vorrichtung nach einem der Ansprüche 11 bis 14, wobei das Bilderzeugungssystem (21) dafür ausgelegt ist, das auf dem Bildschirm (20) angezeigte Bild auf variable Weise als Funktion wenigstens eines Messergebnisses, das zwischen dem Wert des Beobachtungsabstands (DO) und dem Wert des Pupillendurchmessers des Anwenders (10) gewählt wird, zu erzeugen.

**Claims**

**1.** Method for characterizing a blur perceived by a subject (10), comprising the following steps:

/1/ selecting a criterion of perceived blur allowing a blur limit from which the criterion is or is not met for the subject (10) to be established;
/2/ generating then displaying an image (C) on a display screen (20), said image having a blur corresponding to low-pass filtering of image components as a function of spatial frequencies of said image components;
/3/ getting the subject (10), said subject being free to move away from or toward the display screen (20) and to vary in this way a viewing distance (DO), to view the displayed image (C) and to indicate whether the criterion of perceived blur selected in step /1/ is met or not;
/4/ selecting a value of the viewing distance (DO) adopted by the subject (10) at at least one instant in step /3/;
/5/ on the basis of the value selected in step /4/ for the viewing distance (DO), of a cutoff spatial frequency of the low-pass filtering of the displayed image (C) and optionally of a value of pupil diameter, determining a value of a dioptric blur for the displayed image viewed by the subject (10), said value being associated with the indication provided by the subject in step /3/ in response to the criterion of perceived blur,
and said value of the dioptric blur being a decreasing function of the value selected for the viewing distance (DO) when the cutoff spatial frequency of the low-pass filtering of the displayed image (C) is constant.

**2.** Method according to Claim 1, according to which the value of the dioptric blur determined in step /5/ is inversely proportional to the value selected in step /4/ for the viewing distance (DO) when the cutoff spatial frequency of the low-pass filtering of the displayed image (C) is constant, and optionally is furthermore an increasing function of the value of pupil diameter.

**3.** Method according to Claim 1 or 2, according to which the value of the dioptric blur determined in step /5/ is inversely proportional to the cutoff spatial frequency of the low-pass filtering of the displayed image (C) when the value selected in step /4/ for the viewing distance (DO) is constant.

**4.** Method according to any one of the preceding claims, according to which the viewing distance (DO) of the subject (10) from the displayed image (C) is measured continuously in step /3/.

**5.** Method according to any one of Claims 1 to 4, according to which the value selected in step /4/ for the viewing distance (DO) corresponds to the shortest viewing distance adopted by the subject (10) at at least one instant in step /3/.

**6.** Method according to any one of Claims 1 to 4, according to which the value selected in step /4/ for the viewing distance (DO) is indicated by the subject (10) as providing a maximum viewing comfort when the displayed image (C) is viewed by said subject.

**7.** Method according to any one of the preceding claims, according to which the sequence of steps /2/ to /5/ is carried out a plurality of times, the cutoff spatial frequency of the low-pass filtering of the displayed image being varied in successive iterations of said sequence of steps so that the subject (10) provides opposite indications in step /3/ in response to the criterion of perceived blur in at least two iterations of said sequence of steps,
and according to which the bounds of a threshold value of the dioptric blur corresponding to said perceived criterion are deduced for the subject (10) from the opposite indications provided by said subject, which indications are correlated with the dioptric blur values determined during corresponding iterations of step /5/.

**8.** Method according to any one of the preceding claims, according to which the value used for the pupil diameter in step /5/ results from a measurement carried out on the subject (10).

**9.** Method according to Claim 8, according to which the measurement of pupil diameter is carried out on the subject (10) continuously in step /3/, and according to which the value used for said pupil diameter in step /5/ corresponds to the same instant as the viewing distance (DO) selected in step /4/.

**10.** Device able to characterize a blur perceived by a user (10), comprising:

- an image-generating system (21), each image having a blur corresponding to a low-pass filtering of image components as a function of spatial frequencies of said image components;
- a screen (20) able to display the images generated by the image-generating system; and
- a data-processing unit able to determine a value of a dioptric blur on the basis of a value of the viewing distance (DO) adopted by the user (10) to observe an image (C) displayed on the screen (20), of a cutoff spatial frequency of the low-pass filtering of the displayed image, and optionally of a pupil diameter value.

**11.** Device according to Claim 10, furthermore comprising a system for measuring distance, which system is arranged to measure the viewing distance (DO) between the user (10) and the screen (20).

**12.** Device according to Claim 11, in which the system for measuring distance is able to measure the viewing distance (DO) without a continuous material connection between the screen (20) and the user (10), and preferably using a continuous series of measurements.

**13.** Device according to any one of Claims 10 to 12, furthermore comprising a system for measuring a pupil diameter of the user (10).

**14.** Device according to Claim 11 or 12 together with Claim 13, in which the system for measuring distance and the system for measuring pupil diameter are able to measure the viewing distance (DO) and the pupil diameter of the user (10) simultaneously, at a given instant.

**15.** Device according to any one of Claims 11 to 14, in which the image-generating system (21) is able to generate the image displayed on the screen (20) variably as a function of at least one measurement result chosen from the value of the viewing distance (DO) and the value of the pupil diameter of the user (10).

FIG.1.

EP 2 448 465 B1

SELECTION DU MOTIF ── 1

↓

FILTRAGE D'UNE IMAGE NETTE DU MOTIF ── 2

↓

AFFICHAGE DE L'IMAGE FILTREE ── 3

↓

SELECTION DU CRITERE DE PERCEPTION DE FLOU ── 4

↓

OBSERVATION DE L'IMAGE AFFICHEE ── 5

↓

REPONSE DU SUJET ── 6

↓

SELECTION D'UNE VALEUR DE LA DISTANCE D'OBSERVATION ── 7

↓

ATTRIBUTION DE LA VALEUR DE FLOU DIOPTRIQUE ── 8

FIG.2.

**EP 2 448 465 B1**

**Documents brevets cités dans la description**

- US 2004174499 A